# EUROPEAN PATENT APPLICATION

(11) **EP 1 199 670 A2**
(43) Date of publication of application: **24.04.2002**
(21) Application number: 01124315.1
(22) Date of filing: 19.10.2001
(51) Int. Cl.: G06F 19/00

(54) **Medical diagnosis system and diagnosis-processing method thereof**

(30) Priority: 19.10.2000 JP 2000319330
(71) Applicant: NIPRO CORPORATION, Osaka-shi Osaka 531 (JP)
(72) Inventor: Takehito, Ito, Kita-ku, Osaka-shi, Osaka-fu (JP); Toshihiro, Kikuchi, Kita-ku, Osaka-shi, Osaka-fu (JP)
(74) Representative: VOSSIUS & PARTNER

(57) **Abstract**

In a medical diagnosis system, user's medical information received by a communication unit (31) are analyzed by analyzing means (33) with reference to the medical information stored in first data storage means (34). The data-extracting means (36) extracts the result analyzed by the analyzing means(33), the content of doctor's questions from the doctor's questions storage means (35), and information of the user from the first data storage means (34) according to the result analyzed by the analyzing means (33), and determines a physician suitable for the health condition of the user. The result analyzed by the analyzing means(33)or the content of doctor's questions is sent to the user terminal equipment (1) through the communication unit(31). An advice of a specialist to have close examination and the selected physician's name are sent to the user terminal equipment (1) through the communication unit(31). The information of the user and request for consultation are sent to a physician terminal equipment(2).

## Description

The present invention relates to a system and method of medical diagnosis using a communication system and, more particularly, to a medical diagnosis system and a diagnosis-processing method employing the same, particularly suitable for health care of healthy persons.

When treating a patient, it is general practice for a physician to diagnose a patient before medical treatment by information based on doctor's questions such as the presence of rational symptom and life habits, and biological information such as blood pressure, pulsation, body temperature, cardiac sound and the like. For the healthy persons, check-up examination is carried out for health care and serves for early identification and prevention of diseases.

In most companies, a mass health examination is executed annually in spring or autumn. Once a person has lost the opportunity for mass medical examination, he is obliged to visit a clinic or hospital for health examination on a normal business day, causing a troublesome problem. Further, the interval between health examinations is too long to achieve early identification and prevention of diseases.

For home-bound patients or persons having inveterate diseases such as diabetes, cardiopathy or the like, there have been proposed various medical information-communicating systems such as a home medical care system, an doctor's questions system, and an emergency care system (e.g., JP-A H9-101998, JP-A H11-353185, JP-A 2000-139857).

H9-101998 discloses a home health care system intended for home health care of healthy persons, elderly persons or persons who need nursing care. In this system, biological information of a patient is transmitted together with the identification number of the patient to a data processing equipment of a medical treatment center where the biological information is processed to determine whether or not the latest value of the biological information is in the range of predetermined values. If the latest value is judged as being out of the range of predetermined values, the identification number and the values of the biological information transmitted therewith are displayed on a display together with the results of past examinations and the patient record stored in the database.

JP-A H11-353185 discloses an interview system for medical treatment comprising a medical treatment interview knowledge base storage means, an interview processing means, and a medical treatment interview knowledge base editing means, wherein medical treatment interview knowledge base is displayed on a display in the tree structure and is modified to add or delete a question by selecting a node with a mouse.

JP-A 2000-139857 discloses a remote medical data diagnosing device comprising a watch type radio telephone device having the function for measuring blood pressure and the heart rate; a medical treatment center server for automatically collecting and diagnosing measured data sent from the watch type radio telephone device; and an emergency call center for directly establishing contact with a user by means of a telephone when the measured value is out of order, and a customer center for periodically mailing diagnostic results in tabular form to the user.

However, the medical data communication systems are directed to applications for homebound patients or sufferers from diabetes mellitus, cardiac disease or other inveterate disease. There is no medical data communication system intended for early identification and prevention of diseases of healthy persons by their health care administration.

It is therefore an object of the present invention to provide a medical diagnosis system and a diagnosis-processing method thereof, which enables healthy persons to perform any time health care administration for the purpose of prevention and early identification of diseases.

The present invention has been achieved by meeting the situation that simple and careful measures can be taken by use of a diagnostic result based on user's own biological information in combination with doctor's questions selected according to the biological information.

According to the present invention, there is provided a medical diagnosis system comprising:
at least one user terminal equipment;
a center equipment located in a medical center for receiving and processing the medical information sent from said user terminal equipment; and
at least one physician terminal equipment for communicating with said center equipment to exchange the medical information;
said user terminal equipment comprising means for detecting biological information of the user in the form of electric signals, means for monitoring circumstances of detection, means for sending user's medical information including the detected biological information to said center equipment, and means for receiving medical information from the center equipment,
said center equipment comprising first data storage means for storing medical treatment informations about users and information about physicians, data-receiving means for receiving said user's medical information sent from said user terminal equipment, data-analyzing means for analyzing said user's medical information received by said receiving means, means for producing a diagnostic result corresponding to the analyzed result of said user's medical information, second data storage means for storing doctor's questions selectable according to the analyzed result, reading-means for reading selected doctor's questions from said second data storage means, means for selecting at least one physician among said physicians stored in said first data storage means, means for communicating with said user terminal equipment and/or physician terminal equipment to send said medical information including said diagnostic result and said doctor's questions;
said physician terminal equipment comprising data-receiving means for receiving said medical information sent from said center equipment, and data-transmitting means for sending medical information of the physician side to said center equipment.

According to one aspect of the present invention there is provided a diagnostic processing method comprising the steps of analyzing user's medical information sent from a user terminal equipment, and carrying on diagnosis of the user who had been determined as being abnormal but requiring no emergency treatment, by use of a diagnostic result based on said user's biological information in combination with doctor's questions selected on the basis of said analyzed result.

In a preferred embodiment of the present invention, the diagnostic processing method comprises the steps of
analyzing user's medical information sent from a user terminal equipment to determine the health condition of the user categorized into three cases, (a) not particular, (b) abnormal but no need to emergency care, and (c) abnormal and need to emergency care;
sending the determined result to the user in case of the category (a), or sending doctor's questions determined on the basis of said analyzed results to the user to request answers to said doctor's questions in case of the category (b), or sending a request for medical treatment to a suitable physician selected on the basis of said analyzed result in case of the category (c) . In case of the category (b), the method further includes the steps of analyzing received answers to said doctor's questions to determine the health condition of the user categorized into three cases, (a) not particular, (b) abnormal but no need to emergency care, and (c) abnormal and need to emergency care; and sending the determined result along with health advice to the user in case of the category (a), or advising the user to have close examination with a message of introduction of a suitable physician selected on the basis of said determination in case of the category (b), or sending a request for medical treatment to a suitable physician selected on the basis of said determination in case of said category (c).

The present invention will become more fully understood from the detailed description given hereinafter and the accompanying drawings, which are given by way of illustration only. However, it should be understood that the detailed description and specific example, while indicating preferred embodiments of the invention, are given by way of illustration only, since various changes and modifications within the spirit an scope of the invention will become apparent to those skilled in the art from the detailed description.

### BRIEF DESCRIPTION OF THE DRAWINGS

Fig. 1 is a diagram of a health examination system according to the present invention;
Fig. 2 is a flow chart of a diagnostic processing method according to the present invention.
Fig. 3 is a flow chart illustrating a diagnostic process subsequent to that of Fig. 2.

### DETAILED DESCRIPTION OF THE INVENTION

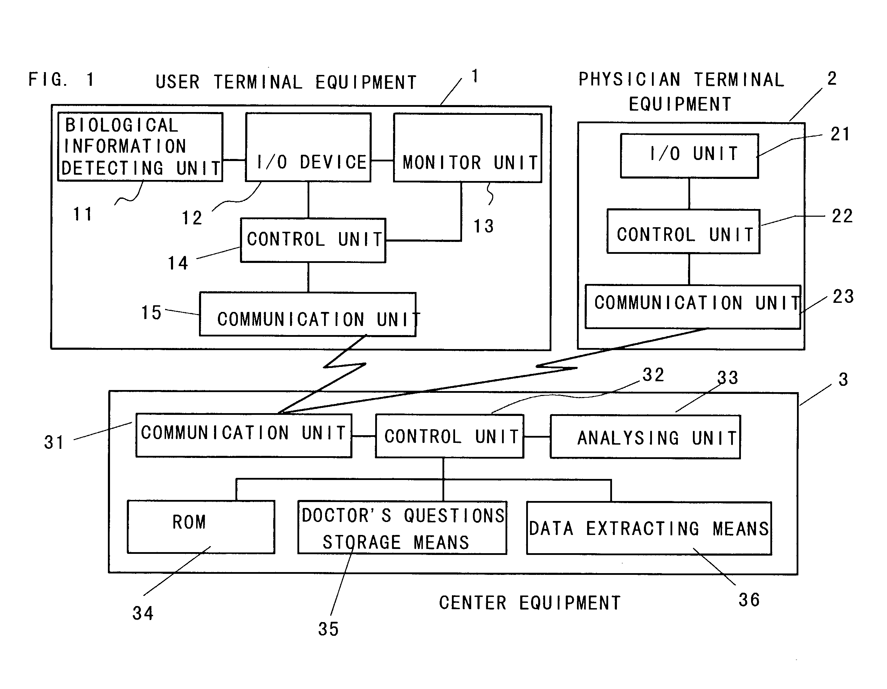

Referring to Fig. 1, there is shown a medical diagnosis system according to the present invention, which comprises at least one user terminal equipment 1 located in a home or an office, at least one physician terminal equipment 2 located in a hospital or climic, and a center equipment 3 located in a medical center, all of which are connected by communication networks.

The user terminal equipment 1 is provided with a biological information-detecting unit 11 (which may be removably connected to the user terminal equipment). The biological information (e.g., blood pressure, a heart rate, body temperature, weight and the like) are detected by the detecting unit 11, automatically input to an input/output unit 12, and displayed on a monitor 13 to allow the user to observe the detected information. A control unit 14 controls the input/output (I/O) unit 12 and monitor 13. If the control unit 14 determines that the biological data are in defective condition, it give a signal to the biological information-detecting unit 11 to repeat the detection of biological data until complete biological data are obtained.

The input/output unit 12 is so constructed that the user's medical information including biological information can be writen therein. The medical information such as answers to the doctor's questions and other medical information, which are unable to be detected by the biological information detecting unit 11, can be writen manually to the input/output unit 12 by the user. The medical information input to the input/output unit 12 is sent to the center equipment 3 by a communication unit 15 through the communication network.

A communication unit 31 of the center equipment 3 receives the medical information from the user terminal equipment 1 through the communication network.

The center equipment 3 comprises a control unit 32 which controls analyzing unit 33, first data storage means such as read-only-memory (ROM) 34, doctor's questions storage means 35 serving as second storage menans, and data-extracting means 36 for extracting contents of the result and doctor's questions. The first data storage means or ROM 34 stores address, age, gender and other informations of each user, and personal histories, area of specialization and other informations of each physician, in additions to the medical information of each user. The medical informations from the user terminal equipment 1 are analyzed by the analyzing means 33 with reference to the medical informations stored in ROM 34, to determine the health condition of the user. The data-extracting means 36 extracts the judged result from the analyzing means 33, the content of doctor's questions from the doctor's questions storage means 35, and information on the user from the ROM 34 according to the result determined by the analyzing means 33, and determines a physician suitable for the health condition of the user. The result determined by the analyzing means 33 or the content of doctor's questions is sent to the user terminal equipment 1 through the communication unit 31. As a result of analysis of answers to the doctor's questions, if the user is judged as being in a condition that does not need emergency care but requires examination by a specialist, advice of close examinations by a specialist and selected physician's name are sent to the user terminal equipment 1 through the communication unit 31. If the user is judged as being in a condition requiring an emergency care, the information on the user and request of examination are sent to the physician terminal equipment 2 through the communication unit 31.

The information of the user received by a communication unit 23 of the physician terminal equipment 2 are input to an input/output unit 21 through a control unit 22. The input/output unit 21 is controlled by the control unit 22 and prevented from redundant input of the information of the user. Further, the input/output unit 21 allows the physician to manually input or output the information. The physician terminal equipment 2 is so designed as to be switched over from attended operation to unattended operation or vice versa. At the time of the unattended operation, the physician terminal equipment 2 may send a reply of refusal to request because of absence to the center equipment 3.

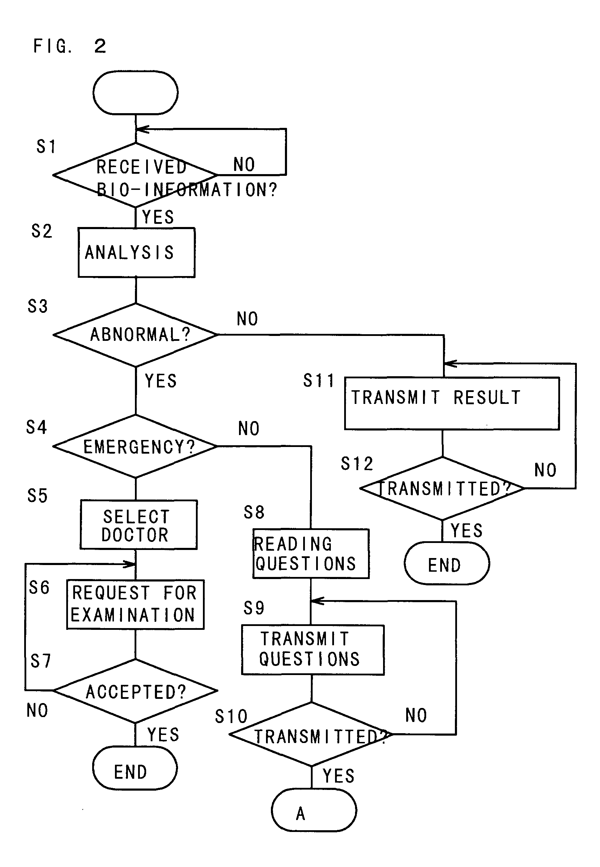

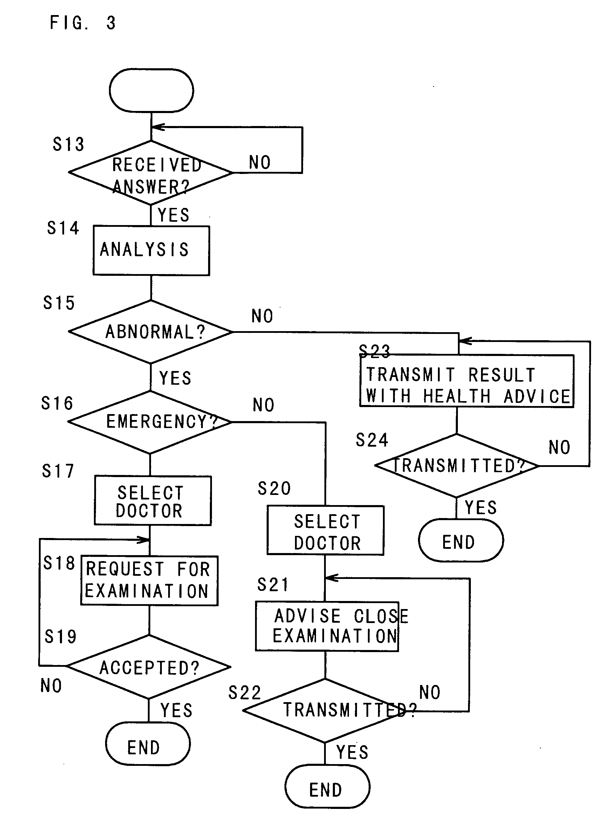

The medical diagnostic processing method of the present invention will be explained below with reference to Figs. 2 and 3.

The medical diagnostic processing method of the present invention comprises the steps of analyzing user's medical information sent from the user terminal equipment, and carrying on diagnosis of the user who had been determined as being abnormal but requiring no emergency treatment, by use of a diagnostic result based on said user's biological information in combination with doctor's questions selected on the basis of said analyzed result.

When the user's biological information are input to the communication unit 31 of the center equipment 3 (S1), the analyzing unit 33 of the center equipment 3 performs an analysis of the biological information (S2). According to the analyzed result, the center equipment 3 makes a judgement as to the existence of abnormality (S3). If no abnormality is recognized, the result of the judgement is sent to the user terminal equipment 1 through the communication unit 31 (S11) and the diagnostic process is terminated after the transmission has been confirmed (S12).

If the presence of abnormality is recognized, an additional judgement is made as to the necessity of emergency treatment (S4). If no necessity of emergency treatment is recognized, the content of doctor's questions is taken out from the doctor's questions storage means 35 according to the analyzed result (S8) and sent to the user terminal equipment 1 through the communication unit 31 (S9). After the transmission has been confirmed (S10), the diagnostic process is terminated.

If the necessity of emergency treatment is recognized, a suitable physician is selected from the ROM 34 according to the analyzed result (S5) and the request for medical examination is sent to the selected physician (S6). At the same time, various information about the user who had been judged as requiring emergency treatment, such as medical information, address, name, age, sex and the like are read out from the ROM 34 and sent to the selected physician. If the return mail accepting the request is received by the center equipment 3 through the communication unit 31 (S7), the diagnostic process is terminated. In this case, there exists an urgent need to examine the user or patient, and thus the transmission is confirmed by the acceptance of the request. If the return mail accepting the request is not received within a certain period of time, e.g., within 1 minute, the request for medical examination is sent again to the same physician. In case of the absence of physician, the physician terminal equipment 2 with unattended operation transmits a return mail notifying refusal of acceptance because of absence. As soon as the center equipment 3 received the notice of refusal, a request for medical examination is sent to another physician secondarily selected.

A method for processing answers to the doctor's questions will be explained below with reference to Fig 3.

When the answers to the doctor's questions are input to the center equipment 3 through the communication unit 31 (S13), the analyzing means 33 performs analysis of the answer (S14). Based on the analyzed result, the center equipment 3 makes a judgement as to the existence of abnormality (S15). If no abnormality is recognized, the result of judgement is sent together with advice for health to the user terminal equipment 1 through the communication unit 31 (S23) and the process is terminated after the transmission has been confirmed (S24).

If the presence of abnormality is recognized, an additional judgement is made as to the necessity of emergency treatment (S16). If no necessity of emergency treatment is recognized, a suitable physician is selected from the registrant stored in the ROM 34 according to the analyzed result (S20) and an advice of close examination is sent to the user together with a message of introduction to the selected physician (S21). After the transmission has been confirmed (S22), the process is terminated.

If the necessity of emergency treatment is recognized, a suitable physician is selected from the registrant stored in the ROM 34 according to the analyzed result (S17) and a request for medical examination is sent to the selected physician (S18). At the same time, various information about the user who had been judged as requiring emergency treatment, for example, medical information, address, name, age, sex of the user are read out from the ROM 34 and sent to the selected physician. If a return mail notifying acceptance of the request is received by the center equipment 3 (S19), the process is terminated. Even in that case, the transmission is confirmed by the acceptance of the request since there exists an urgent need to examine the user or patient.

As will be understood from the above explanation, the present invention allows the user to take medical advice from a doctor on demand, thus making it possible to perform early detection and prevention of diseases effectively.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

## Claims

1. A medical diagnosis system comprising at least one user terminal equipment, a center equipment located in a medical center for receiving and processing the medical information sent from said user terminal equipment, and at least one physician terminal equipment for communicating with said center equipment to exchange the medical information;
said user terminal equipment comprising means for detecting biological information of the user in the form of electric signals, means for monitoring circumstances of detection, means for sending user's medical information including the detected biological information to said center equipment, and means for receiving medical information from the center equipment;
said center equipment comprising first data storage means for storing medical treatment informations about users and information about physicians, data-receiving means for receiving said user's medical information sent from said user terminal equipment, data-analyzing means for analyzing said user's medical information received by said receiving means, means for producing a diagnostic result corresponding to the analyzed result of said user's medical information, second data storage means for storing doctor's questions selectable according to the analyzed result, reading-means for reading selected doctor's questions from said second data storage means, means for selecting at least one physician among said physicians stored in said first data storage means, means for communicating with said user terminal equipment and/or physician terminal equipment to send said medical information including said diagnostic result and said doctor's questions;
said physician terminal equipment comprising data-receiving means for receiving said medical information sent from said center equipment, and data-transmitting means for sending medical information of the physician side to said center equipment.

2. A diagnostic processing method comprising the steps of analyzing user's medical information sent from a user terminal equipment, and carrying on diagnosis of the user who had been determined as being abnormal but requiring no emergency treatment, by use of a diagnostic result based on said biologycal information in combination with doctor's questions selected on the basis of said analyzed result.

3. The diagnostic processing method according to claim 2, further comprising the steps of
analyzing user's medical information sent from a user terminal equipment to determine the health condition of the user categorized into three cases, (a) not particular, (b) abnormal but no need to emergency care, and (c) abnormal and need to emergency care;
sending the determined result to the user in case of the category (a), or sending doctor's questions determined on the basis of said analyzed results to the user to request answers to said doctor's questions in case of the category (b), or sending a request for medical treatment to a suitable physician selected on the basis of said analyzed result in case of the category (c);
wherein, in case of the category (b), said method further including the steps of analyzing received answers to said doctor's questions to determine the health condition of the user categorized into three cases, (a) not particular, (b) abnormal but no need to emergency care, and (c) abnormal and need to emergency care; and sending the determined result along with health advice to the user in case of the category (a) , or sending an advice of close examination to the user along with a message of introduction of a suitable physician selected on the basis of said determination in case of the category (b), or sending a request for medical treatment to a suitable physician selected on the basis of said determination in case of said category (c).
